(19) Europäisches Patentamt
European Patent Office
Office européen des brevets

(11) Veröffentlichungsnummer: **0 130 945**
**B1**

(12) # EUROPÄISCHE PATENTSCHRIFT

(45) Veröffentlichungstag der Patentschrifft: **17.01.90**

(51) Int. Cl.⁴: **C 07 D 401/04**, C 08 K 5/34

(21) Anmeldenummer: **84810309.9**

(22) Anmeldetag: **25.06.84**

(54) **N-Piperidyl-lactame als Lichtschutzmittel.**

(30) Priorität: **30.06.83 US 509102**
**21.10.83 US 544309**

(43) Veröffentlichungstag der Anmeldung:
**09.01.85 Patentblatt 85/02**

(45) Bekanntmachung des Hinweises auf die Patenterteilung:
**17.01.90 Patentblatt 90/03**

(84) Bennante Vertragsstaaten:
**DE FR GB IT**

(56) Entgegenhaltungen:
**EP-A-0 020 293**
**EP-A-0 034 805**
**EP-A-0 034 829**
**DE-A-2 040 975**
**FR-A-2 194 748**

(73) Patentinhaber: **CIBA-GEIGY AG**
**Klybeckstrasse 141**
**CH-4002 Basel (CH)**

(72) Erfinder: **Malherbe, Roger Franfois, Dr.**
**Bleichestrasse 7**
**CH-4058 Basel (CH)**

EP 0 130 945 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die Erfindung betrifft Verbindungen, die in ihrem Molekül sowohl eine Lactamgruppe wie eine sterisch gehinderte Piperidingruppe enthalten und als Stabilisatoren für organisches Material gegen Schädigung durch Licht und Hitze verwendbar sind, sowie das so stabilisierte Material.

Sterisch gehinderte Amine mit einer 2,2,6,6-Tetraalkylpiperidin-Struktur sind als wirkungsvolle Lichtschutzmittel für organisches Material seit längerem bekannt und haben als solche erhebliche kommerzielle Bedeutung erlangt. Eine allgemeine Beschreibung solcher gehinderter Amine haben H. J. Heller und H. R. Blattmann in Pure & Applied Chemistry *36* (1975) 141 - 161 gegeben.

Aus US-3 850 877 und 4 033 928 ist bekannt, dass Ester und Amide von 2,2,6,6-Tetramethylpiperidinen gute Lichtschutzmittel für polymere Substrate, insbesondere für Polyolefine und Epoxidharze sind. Das US-4 309 546 beschreibt N-Piperidin-4'-yl-2-pyrrolidon-4-carbonsäuren und deren Derivate der Formel

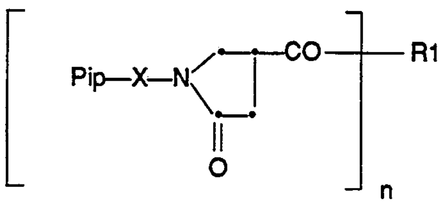

worin n 1 - 4 ist, $R_1$ eine Hydroxyl-, Alkoxy-, Amino- oder substituierte Aminogruppe ist, X eine direkte Bindung oder eine bindende Gruppe ist und Pip eine gegebenenfalls substituierte 2,2,6,6-Tetraalkylpiperidin-4-yl-Gruppe ist. Auch diese Verbindungen sind als Lichtschutzmittel verwendbar.

Die vorliegende Erfindung beschreibt Verbindungen, die den zuletzt genannten strukturell verwandt sind, jedoch eine bessere Löslichkeit und eine erhöhte Hydrolysenstabilität besitzen. Es handelt sich dabei um Verbindungen; bei denen eine sterisch gehinderte Piperidingruppe in 4-Stellung mit einem Lactam verknüpft ist und die als Lichtschutzmittel für organische Polymere verwendbar sind.

Die Erfindung betrifft Verbindungen der Formel I oder II

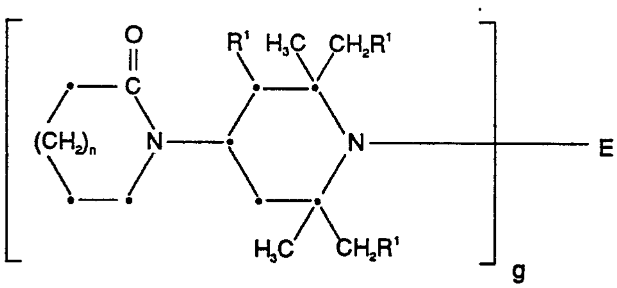

(I)

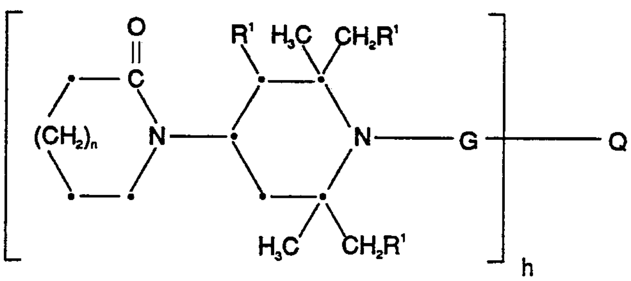

(II)

worin n 0, 1, 2 oder 3 ist, g 1 oder 2 ist, h eine Zahl von 1 bis 4 ist,

$R^1$ Wasserstoff oder $C_1$-$C_5$-Alkyl bedeutet,

E, wenn g 1 ist, H, $O^\cdot$, OH, $C_1$-$C_{12}$-Alkyl, $C_3$-$C_8$-Alkenyl, Propargyl, Benzyl, CN, $C_2$-$C_4$-Hydroxyalkyl, $C_2$-$C_{10}$-Alkanoyl, $C_3$ - $C_7$ Alkenoyl, Benzoyl, $C_2$-$C_8$-Alkoxy, $C_2C_{10}$-Alkanoyloxy, $C_3$-$C_4$-Alkenoyloxy oder Benzoyloxy bedeutet, und wenn g 2 ist, unverzweigtes oder verzweigtes $C_2$-$C_6$-Alkylen, Phenylethylen(Styrolen), 2-Butenylen oder $C_8$ - $C_{15}$ Phenylendialkylen bedeutet,

G ein Rest der Formel $-CH_2-CH(R^2)-O-$ oder $-CH_2-COO-$ ist, dessen Methylengruppe an den Piperidin-Stickstoff und dessen Sauerstoff an Q gebunden ist und worin $R^2$ Wasserstoff, Methyl, Ethyl oder Phenyl bedeutet, und

Q, wenn G $-CH_2-CH(R^3)-O-$ ist,
im Falle von h = 1 eine $C_2$-$C_{10}$-Alkanoylgruppe, Benzoylgruppe oder eine Gruppe der Formel III

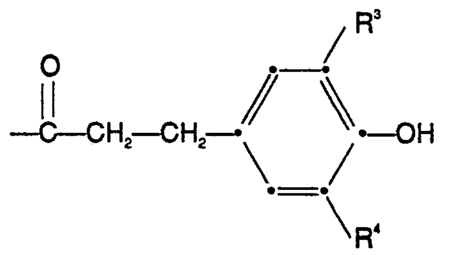

(III)

bedeutet, worin $R^3$ $C_1$-$C_8$-Alkyl und $R^4$ verzweigtes $C_3$-$C_8$-Alkyl bedeuten,
im Falle von h = 2 -CO-, -COCO- oder $C_3$-$C_{12}$-Alkandicarbonyl bedeutet,
im Falle von h = 3 $C_6$-$C_{10}$-Alkantricarbonyl bedeutet und
im Falle von h = 4 $C_8$-$C_{12}$-Alkantetracarbonyl bedeutet und,
wenn G $-CH_2-COO-$ ist,

Q im Falle von h = 1 $C_1$-$C_{12}$-Alkyl oder eine Gruppe der Formel IV

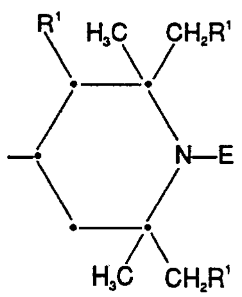

(IV)

bedeutet
im Falle von h = 2 $C_2$-$C_{12}$-Alkylen, $C_6$-$C_8$-Cycloalkylen, 3-Oxapentamethylen, 1,4-Cyclohexylen-dimethylen oder eine Gruppe der Formel V

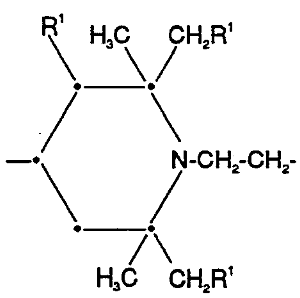

(V)

bedeutet,
im Falle von h = 3 $C_3$-$C_8$-Alkantriyl bedeutet und
im Falle von h = 4 $C_4$-$C_{10}$-Alkantetrayl bedeutet.

Die erfindungsgemässen Verbindungen haben also mindestens eine Lactamgruppe. Wenn in dieser Gruppe n = O ist, so handelt es sich um eine Pyrrolidongruppe. Wenn n = 1 ist, so handelt es sich um eine Delta-Valerolactamgruppe, wenn n = 2 ist, um eine Epsilon-Caprolactamgruppe und wenn n = 3 ist, um eine Zeta-Önantholactamgruppe. Bevorzugt sind die Verbindungen mit n = 2 oder 3, insbesondere mit n = 2.

Bevorzugt sind weiterhin Verbindungen der Formel I oder II, worin $R^1$ Wasserstoff ist.

Wenn in Formel I g = 1 ist, kann E unverzweigtes oder verzweigtes $C_1$-$C_{12}$-Alkyl sein, wie z. B. Methyl,

Ethyl, Propyl, Isopropyl, n-Butyl, sec.-Butyl, n-Amyl, Isoamyl, n-Hexyl, n-Octyl, 2-Ethylhexyl, n-Decyl oder n-Dodecyl, vorzugsweise $C_2$-$C_4$-Alkyl. E als $C_3$-$C_8$-Alkenyl kann z. B. Allyl, Methallyl, 2-Butenyl (Crotyl), Hexenyl oder Octenyl, vorzugsweise jedoch Allyl.

E als Hydroxyalkyl kann z. B. 2-Hydroxyethyl, 2-Hydroxypropyl oder 2-Hydroxybutyl sein, vorzugsweise 2-Hydroxyethyl.

E als $C_2$-$C_{10}$-Alkanoyl kann z. B. Acetyl, Propionyl, Butanoyl, Valeroyl, Caproyl, Capryloyl oder Decanoyl sein, vorzugsweise Acetyl. E als $C_3$-$C_4$-Alkanoyl kann z. B. Acryloyl, Methacryloyl oder Crotonoyl sein. E als $C_2$-$C_8$-Alkoxy kann z. B. Methoxy, Ethoxy, Isopropoxy, n-Butoxy, Hexyloxy oder Octyloxy sein. E als $C_2$-$C_{10}$-Alkanoyloxy kann z. B. Acetoxy, Propionyloxy, Butanoyloxy, Valeroyloxy, Octanoyloxy oder Decanoyloxy sein. E als Alkanoyloxy kann z. B. Acryloxy, Methacryloxy oder Crotonyloxy sein.

Vorzugsweise ist E nur im Falle von g = 1 Wasserstoff, O·, OH, $C_1$-$C_4$-Alkyl, Allyl, 2-Hydroxyethyl, Acetyl, Propargyl, Benzyl, CN, Benzoyl oder Benzoyloxy.

Wenn in Foreml I g = 2 ist, kann E unverzweigtes oder verzweigtes $C_2$-$C_6$-Alkylen sein, wie z. B. 1,2-Ethylen, 1,2-Propylen, 1,2-Butylen, Tri-, Tetra-, Penta- oder Hexamethylen.

Vorzugsweise ist E als Alkylen 1,2-Ethylen. E als Phenylendialkylen kann z. B. m- oder p-Xylylen oder p-Phenylen-diethylen sein. Vorzugsweise ist E im Falle von g = 2 1,2-Ethylen oder p-Xylylen.

Wenn in Formel II G ein Rest-$CH_2$-$CH(R^2)$-O- ist und h = 1 ist, kann Q $C_2$-$C_{10}$-Alkanoyl sein, wie z. B. Acetyl, Propionyl, Butanoyl, Valeroyl, Caproyl, Capryloyl oder Decanoyl, vorzugsweise Acetyl. Wenn Q eine Gruppe der Formel III ist, so ist darin $R^3$ $C_1$-$C_8$-Alkyl, und $R^4$ ist darin verzweigtes $C_3$-$C_8$-Alkyl, wie z. B. Isopropyl, tert.-Butyl, tert.-Amyl oder tert.-Octyl. Vorzugsweise sind $R^3$ und $R^4$ tert.-Butyl.

Wenn h = 2 ist, kann Q $C_3$-$C_{12}$-Alkandicarbonyl bedeuten, wie z. B. Malonyl, Succinyl, Adipoyl, Suberoyl, Sebacoyl oder Decan-1,10-dicarbonyl. Vorzugsweise bedeutet in diesem Falle Q eine $C_6$-$C_{10}$-Alkandicarbonylgruppe.

Wenn h = 3 ist, kann Q $C_6$-$C_{10}$-Alkantricarbonyl bedeuten, wie z. B. Tricarballyl oder Butan-1,2,4-tricarbonsäure. Wenn h = 3 ist, kann Q $C_8$-$C_{12}$-Alkantetracarbonyl bedeuten, wie z. B. Butan- 1,2,3,4-tetracarbonyl.

Bevorzugt sind Verbindungen der Formel II, worin G ein Rest der Formel -$CH_2$-$CH_2$-O- ist, h = 1 ist und Q Acetyl oder eine Gruppe der Formel III ist, worin $R^3$ und $R^4$ tert.-Butyl sind, oder h = 2 ist und Q ein $C_6$-$C_{10}$-Alkandicarbonylrest ist.

Wenn in Formel II G ein Rest -$CH_2$-COO- ist und h = 1 ist, so kann Q $C_2$-$C_{12}$-Alkyl bedeuten und kann dabei unverzweigt oder verzweigt sein, so wie E in Formel I, worin g = 1 ist.

Im Falle von h = 2 kann Q $C_2$-$C_{12}$-Alkylen sein, wie z. B. 1,2-Ethylen, Tri-, Tetra-, Penta-, Hexa-, Octa-, Deca- oder Dodecamethylen. Q kann in diesem Fall auch $C_6$-$C_8$-Cycloalkylen sein, wie z. B. 1,2-, 1,3- oder 1,4-Cyclohexylen oder 1,4-Cyclooctylen. Wenn h = 3 ist, bedeutet Q einen $C_3$-$C_8$-Alkantriylrest, wie z. B. Propan-1,2,3-triyl, Butan-1,2,4-triyl- oder einen Rest der Formel VI

```
       CH2 –
      /
R5 – C – CH2 –                (VI)
      \
       CH2 –
```

worin $R^5$ Methyl, Ethyl oder Propyl ist.

Wenn h = 4 ist, bedeutet Q einen Alkantetraylrest, wie z. B. einen Rest der Formel VI, worin $R^5$ -$CH_2$- ist.

Bevorzugt sind Verbindungen der Formel II, worin G ein Rest der Formel -$CH_2$-COO- ist, h = 1 ist und Q $C_1$-$C_4$-Alkyl oder eine Gruppe der Formel IV ist, oder h = 2 ist und Q $C_2$-$C_8$-Alkylen oder eine Gruppe der Formel V ist.

Die Herstellung der Verbindungen der Formel I und II kann durch Reaktion von Caprolacton mit den entsprechenden 4-Amino-2,2,6,6-tetraalkylpiperidinen nach dem Verfahren des US Patent 3 000 800 geschehen. Dieses Verfahren erfordert jedoch hohe Drucke und Temperaturen.

Besser geeignet ist die folgende Reaktionsfolge, die von den 4-Ketopiperidinen VII ausgeht, welche in Gegenwart einer Aminocarbonsäure VIII hydriert werden. Als Katalysator eignen sich hierzu die üblichen Hydrierkatalysatoren aus Edelmetallen, insbesondere Platin-Katalysatoren. Die entstehenden Piperidin4-ylaminocarbonsäuren IX werden dann durch Erhitzen auf 250 - 300°C, insbesondere 260 - 270°C, unter Stickstoff zu den Lactamen der Formel Ia cyclisiert:

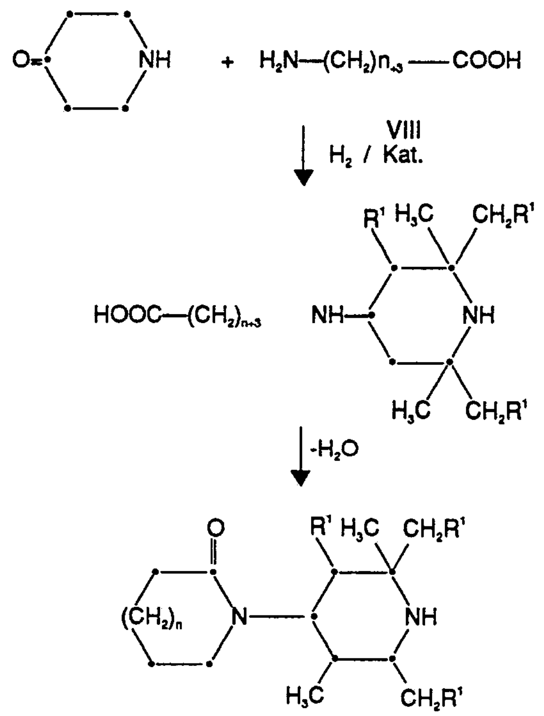


(VII)

(IX)

(Ia)

Aus den Verbindungen der Formel Ia können die Verbindungen der Formel I durch entsprechende N-Substitution hergestellt worden. Hierzu können die für die H-Substitution sekundären Amine üblichen Methoden verwendet werden. Dazu zählt vor allem die Umsetzung mit den Halogenverbindungen E-(Hal) g, worin Hal Chlor, Brom oder Jod ist. Die N-Hydroxyalkyl-Derivate können durch Umsetzung mit Alkylenoxiden hergestellt werden. Die N-Oxyle (E = O·) können durch Oxydation der NH-Verbindungen mit Perverbindungen hergestellt werden und aus den N-Oxylen können durch Reduktion die N-Hydroxylverbindungen (E = OH) hergestellt werden. Letztere können durch Umsetzung mit Carbonsäurechloriden oder -anhydriden in die N-Alkanoyloxy-, N-Alkenoyloxy- und N-Benzoyloxyverbindungen überführt werden. Aus den N-Hydroxyalkylverbindungen (Formel I, g = 1, E = $-CH_2-CH(R^2)-OH$) können durch Versetzung mit einer Mono- oder Polycarbonsäure die Verbindungen der Formel II mit G = $-CH_2-CH(R^2)-O-$ hergestellt werden.

Die Verbindungen der Formel II, worin G $-CH_2COO-$ bedeutet, können hergestellt werden aus Verbindungen der Formel Ia durch Umsetzung mit Chloressigsäureestern der Formel $[ClCH_2-COO-]$ Q. Diese Verbindungen können auch durch Umsetzung in andere Verbindungen der Formel II umgewandelt werden.

Die Verbindungen der Formel I und II sind wirkungsvolle Stabilisatoren für lichtempfindliche organische Materialien gegen deren Schädigung durch Licht, insbesondere durch kurzwelliges Licht. In gewissem Ausmass bewirken sie auch eine Stabilisierung gegen thermische Schädigung. Beispiele für zu stabilisierende organische Materialien sind Öle, Fette und andere kosmetische Grundstoffe, photographische Materialien wie Photofilme oder Photopapiere, insbesondere aber organische Polymere. Zu den Polymeren, die lichtempfindlich sind und sich mit Verbindungen der Formel I und II stabilisieren lassen, gehören:

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z. B. von Cyclopenten oder Norbornen.

2. Mischungen der unter 1) genannten Polymeren, z. B. Mischungen von Polypropylen mit Polyisobutylen.

3. Copolymere von Mono- und Diolefinen untereinander oder mit anderen Vinylmonomeren, wie z. B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z. B. Styrol-Butadien, Styrol-Acrylnitril, Styrol-Alkylmethacrylat, Styrol-Maleinanhydrid, Styrol-Acrylnitril-Methylacrylat; Mischungen von hoher Schlagzähigkeit aus Styrol-Copolymeren und einem anderen Polymer wie z. B. einem Polyacrylat, einem Dien-

Polymeren oder einem Ethylen-Propylen-Dien-Terpolymeren; sowie Block-Copolymere des Styrols, wie z. B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z. B. Styrol auf Polybutaiden, Styrol und Acrylnitril auf Polybutadien, Styrol und Maleinsäureanhydrid auf Polybutadien, Styrol und Alkylacrylate bzw. Alkyl-methacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z. B. als sogenannte ABS, MBS, ASA oder AES-Polymere bekannt sind.

7. Halogenhaltige Polymere, wie z. B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z. B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z. B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z. B. Ethylenoxid enthalten.

13. Polyphenyloxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Block-Copolymere mit Polyethern wie z. B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-di-methylolcyclohexanterephthalat, Polyhydroxybenzoate, sowie Block-Polyether-ester, die sich von Polyethern mit Hydroxylendgruppen ableiten.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie PhenolFormaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z. B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten, oder

Epoxidharzen vernetzt sind.

25. Vernetzte Epoxidharze, die sich von Polyepoxiden ableiten, z. B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren wie z. B. PP/EPDM, Polyamid 6/EPDM oder ABS, PVC/EVA, PVC/ABS, PVC/MBS, PC/ABS, PBTP/ABS.

Von besonderer Bedeutung ist die Licht-Stabilisierung von Polyolefinen, Styrolpolymeren, Polyamiden und Polyurethanen und die erfindungsgemässen Stabilisatoren der Formel I und II sind hierfür besonders geeignet. Beispiele hierfür sind Polyethylen hoher und niederer Dichte, Polypropylen, Ethylen/Propylen-Copolymere, Polyetyrol, Styrol/Butadien/Acrylnitril-Terpolymere, Mischungen von Polyolefinen oder von Styrol-Polymeren sowie Polyurethane auf Polyether- oder Polyester-Basis in Form von Lacken, Fasern, Filmen, Folien, Elastomeren oder Schäumen.

Die Stabilisatoren der Formel I und II werden den Kunststoffen in einer Konzentration von 0,05 bis 5 Gew.-%, vorzugsweise 0,1 bis 2,5 Gew.-% zugegeben. Die Zugabe kann während oder nach der Herstellung der Polymeren erfolgen, beispielsweise durch Zumischen, gegebenenfalls zusammen mit anderen Additionen, vor oder während der Formgebung des Kunststoffes, oder auch durch Aufbringen als Lösung auf das Polymer-Granulat und anschliessendes Verdampen des Lösungsmittels.

Die Stabilisatoren können dem Kunststoff auch als Masterbatch zugegeben werden, der diese Verbindungen in einer Konzentration von etwa 2,5 bis 25 % enthält.

Obwohl die Verbindungen der Formel I und II bereits allein eine ausgezeichnete Lichtschutzwirkung bewirken, kann man sie auch kombinieren mit anderen Stabilisatoren und auch mit anderen Lichtschutzmitteln. So können sie z. B. kombiniert werden mit phenolischen Antioxydantien, Pigmenten, Farbstoffen, Metalldesaktivatoren, UV-Absorbern oder anderen Stabilisatoren. Solche Sostabilisatoren werden üblicherweise ebenfalls in einer Menge von 0,05 bis 5 Gew.-%, vorzugsweise 0,1 bis 2,5-Gew.-% eingesetzt.

Beispiele für mitverwendbare Stabilisatoren sind die folgenden:

**1. Antioxidantien**
**1.1. Alkylierte Monophenole**

2,6-Di-tert.-butyl-4-methylphenol
2-Tert.-butyl-4,6-dimethylphenol
2,6-Di-tert.-butyl-4-ethylphenol
2,6-Di-tert.-butyl-4-n-butylphenol
2,6-Di-tert.-butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.-butyl-4-methoxymethylphenol

**1.2. Alkylierte Hydrochinone**

2,6-Di-tert.-butyl-4-methoxyphenol
2,5-Di-tert.-butyl-hydrochinon
2,5-Di-tert.-amyl-hydrochinon
2,6-Diphenyl-4-octadecyloxyphenol

**1.3. Hydroxylierte Thiodiphenylether**

2,2'-Thio-bis-(6-tert.-butyl-4-methylphenol)
2,2'-Thio-bis-(4-octylphenol)
4,4'-Thio-bis-(6-tert.-butyl-3-methylphenol)
4,4'-Thio-bis-(6-tert.-butyl-2-methylphenol)

**1.4. Alkyliden-Bisphenole**

2,2'-Methylen-bis-(6-tert.-butyl-4-methylphenol)
2,2'-Methylen-bis-(6-tert.-butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)

2,2'-Methylen-bis-(4,6-di-tert.-butylphenol)
2,2'-Ethyliden-bis-(4,6-di-tert.-butylphenol)
2,2'-Ethyliden-bis-(6-tert.-butyl-4-isobutylphenol)
2,2'Methylen-bis-[6-(α-methylbenzyl)-4-nonylphenol]
2,2'Methylen-bis-[6-(α,α-dimethylbenzyl)-4-nonylphenol]
4,4'-Methylen-bis-(2,6-di-tert.-butylphenol)
4,4'-Methylen-bis-(6-tert.-butyl-2-methylphenol)
1,1-Bis-(5-tert.-butyl-4-hydroxy-2-methylphenyl)-butan
2,6-Di-(3-tert.-butyl-5-methyl-2-hydroxybenzyl)-4-methylphenol
1,1,3-Tris-(5-tert.-butyl-4-hydroxy-2-methylphenyl)-butan
1,1-Bis-(5-tert.-butyl-4-hydroxy-2-methylphenyl)-3-n-dodecylmercaptobutan
Ethylenglycol-bis-[3,3-bis-(3'-tert.-butyl-4'-hydroxyphenyl)-butyrat] Di-(3-tert.-butyl-4-hydroxy-5-methylphenyl)-dicyclopentadienDi-[2-(3'-tert.-butyl-2'-hydroxy-5'-methyl-benzyl)6-tert.-butyl-4-methyl-phenyl]-terephthalat.

**1.5 Benzylverbindungen**

1,3,5-Tri-(3,5-di-tert.-butyl-4-hydroxybenzyl)-2,4,6-trimethylbenzolDi-(3,5-di-tert.-butyl-4-hydroxybenzyl)-sulfid
3'5-di-tert.-butyl-4-hydroxybenzyl-mercaptoessigsäure-isooctylester Bis-(4-tert.-butyl-3-hydroxy-2,6-dimethylbenzyl)dithiol-terephthalat
1,3,5-Tris-(3,5-di-tert.-butyl-4-hydroxybenzyl)-isocyanurat
1,3,5-Tris-(4-tert.-butyl-3-hydroxy-2,6-dimethylbenzyl)-isocyanurat
3,5-Di-tert.-butyl-4-hydroxybenzyl-phosphonsäure-dioctadecylester
3,5-Di-tert.-butyl-4-hydroxybenzyl-phosphonsäure-monoethylester, Calcium-salz.

**1.6. Acylaminophenole**

4-Hydroxy-laurinsäureanilid
4-Hydroxy-stearinsäureanilid
2,4-Bis-octylmercapto-6-(3,5-di-tert.-butyl-4-hydroxyanilino)-s-triazin
N-(3,5-di-tert.-butyl-4-hydroxyphenyl)-carbaminsäureoctylester.

**1.7. Ester der β-(3,5-Di-tert.-butyl-4-hydroxyphenyl)propionsäure** mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentärythrit |
| Neopentylglycol | Tris-hydroxyethyl-isocyanurat |
| Thiodiethylenglycol | Di-hydroxyethyl-oxalsäurediamid |

**1.8. Ester der β-(5-tert.-butyl-4-hydroxy-3-methylphenyl)-propionsäure** mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentärythrit |
| Neopentylglycol | Tris-hydroxyethyl-isocyanurat |
| Thiodiethylenglycol | Di-hydroxyethyl-oxalsäurediamid |

**1.9. Amide der β-(3,5-Di-tert.-butyl-4-hydroxyphenyl)-propionsäure**, wie z. B.

N,N'-Di-(3,5-di-tert.-butyl-4-hydroxyphenylpropionyl)-hexamethylendiamin
N,N'-Di-(3,5-di-tert.-butyl-4-hydroxyphenylpropionyl)-trimethylendiamin
N,N'-Di-(3,5-di-tert.-butyl-4-hydroxyphenylpropionyl)-hydrazin

**2. UV-Absorber und Lichtschutzmittel**

**2.1. 2-(2'-Hydroxyphenyl)-benztriazole,** wie z. B. das 5'-Methyl-, 3',5'-Di-tert.-butyl-, 5'-Tert.-butyl-, 5'-(1,1,3,3-Tetramethylbutyl)-,5-Chlor-3,5'-di-tert.-butyl-,5-Chlor-3'-tert.-butyl-5'-methyl-,3'-sec.-Butyl-5'-tert.-butyl,4'-Octoxy-,3',5'-Di-tert.-amyl-, 3',5'-Bis-(α,α-dimethylbenzyl)-Derivat.

**2.2. 2-Hydroxybenzophenone,** wie z. B. das 4-Hydroxy-, 4-Methoxy-, 4-Octoxy-, 4-Decyloxy-, 4-Dodecyloxy-, 4-Benzyloxy-,4,2',4'-Trihydroxy-,2'-Hydroxy-4,4'-dimethoxy-Derivat.

**2.3. Ester von gegebenenfalls substituierten Benzoesäuren,** wie z. B. 4-Tert.-butyl-phenylsalicylat, Phenylsalicylat, Octylphenylsalicylat, Dibenzoylresorcin, Bis-(4-tert.-butylbenzoyl)-resorcin, Benzoylresorcin, 3,5-Di-tert.-butyl-4-hydroxybenzoesäure-2,4-ditert.-butylphenylester,3'5-Di-tert.-butyl-4-hydroxybenzoesäurehexadecylester.

**2.4. Acrylate,** wie z. B. α-Cyan-β,β-diphenylacrylsäure-ethylester bzw.-isooctylester, α-Carbomethoxy-zimtsäuremethylester, α-Cyano-β-methyl-p-methoxy-zimtsäuremethylester bzw.-butylester, α-Carbomethoxy-p-methoxy-zimtsäure-methylester, N-(β-Carbomethoxy-β-cyanovinyl)-2-methyl-indolin,

**2.5. Nickelverbindungen,** wie z. B. Nickelkomplexe des 2,2'-Thio-bis[4-(1,1,3,3-tetramethylbutyl)-phenols], wie der 1 : 1- oder der 1 : 2-Komplex, gegebenenfalls mit zusätzlichen Liganden wie n-Butylamin, Triethanolamin oder N-Cyclohexyl-diethanolamin, Nickeldibutyldithiocarbamat, Nickelsalze von 4-Hydroxy-3,5-di-tert.-butylbenzylphosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

**2.6. Sterisch gehinderte Amine,** wie z. B. Bis-(2,2,6,6-tetramethylpiperidyl)-sebacat
Bis-(1,2,2,6,6-pentamethylpiperidyl)-sebacat
n-Butyl-3,5-di-tert.-butyl-4-hydroxybenzyl-malonsäure-bis (1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-(2,2,6,6-Tetramethyl-4-piperidyl)hexamethylendiamin und 4-tert.-Octylamino-2,6-dichlor-1,3,5-s-triazin,
Tris-(2,2,6,6-tetramethyl-4-piperidyl)-nitrilotriacetat, Tetrakis-(2,2,6,6-tetramethyl-4-piperidyl)-1,2,3,4-butantetracarbonsäure, 1,1'-(1,2-Ethandiyl)-bis-(3,3,5,5-tetramethyl-piperazinon).

**2.7. Oxalsäurediamide,** wie z. B. 4,4'-Di-octyloxy-oxanilid, 2,2'-Di-octyloxy-5,5'-di-tert.-butyl-oxanilid, 2,2'-Di-dodecyloxy-5,5'-di-tert.-butyl-oxanilid, 2-Ethoxy-2'-ethyl-oxanilid, N,N'-Bis-(3-dimethylaminopropyl)-oxalamid, 2-Ethoxy-5-tert.-butyl-2'-ethyloxanilid und dessen Gemisch mit 2-Ethoxy-2'-ethyl-5,4'-di-tert.-butyl-oxanilid, Gemische von ortho- und para-Methoxy- sowie von o- und p-Ethoxy-di-substituierte Oxaniliden.

**3. Metalldesaktivatoren,** wie z. B. N,N'-Diphenyloxalsäurediamid, N-Salicylal-N'-salicyloylhydrazin, N,N'-Bis-salicyloylhydrazin, N,N'-Bis-(3,5-di-tert.-butyl-4-hydroxyphenylpropionyl)-hydrazin, 3-Salicyloylamino-1,2,4-triazol, Bis-benzylidenoxalsäuredihydrazid.

**4. Phosphite und Phosphonite,** wie z. B. Triphenylphosphit, Diphenylalkylphosphite, Phenyldialkylphosphite, Tri-(nonylphenyl)-phosphit, Trilaurylphosphit, Trioctadecylphosphit, Distearyl-pentärythritdiphosphit, Tris-(2,4-di-tert.-butylphenyl)-phosphit, Diisodecylpentaerythrit-diphosphit, Di-(2,4-di-tert.-butylphenyl)-pentärythritdiphosphit, Tristearyl-sorbit-triphosphit,Tetrakis-(2,4-di-tert.-butylphenyl)-4,4'-biphenylen-diphosphonit.

**5. Peroxidzerstörende Verbindungen,** wie z. B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentärythrit-tetrakis-(β-dodecylmercapto)-propionat.

**6. Polyamidstabilisatoren,** wie z. B. Kupfersalze in Kombination mit Jodiden und/oder Phosphorverbindungen und Salze des zweiwertigen Mangans.

**7. Basische Co-Stabilisatoren,** wie z. B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

**8. Nukleierungsmittel,** wie z. B. 4-tert.-Butylbenzoesäure, Adipinsäure, Diphenylessigsäure.

**9. Füllstoffe und Verstärkungsmittel,** wie z. B. Calciumcarbonat, Silikate, Glasfasern, Asbest, Talk, Kaolin, Glimmer, Bariumsulfat, Metalloxide und -hydroxide, Russ, Graphit.

**10. Sonstige Zusätze,** wie z. B. Weichmacher, Gleitmittel, Emulgatoren, Pigmente, Optische Aufheller, Flammschutzmittel, Antistatika, Treibmittel.

Weitere Additive, die mitverwendet werden können, sind z. B. Thiosynergreten, wie Thiodipropionsäureester, Gleitmittel wie Stearylalkohole, Füllstoffe, Asbest, Kaolin, Talk, Glasfasern, Pigmente, optische Aufheller, Flammschutzmittel oder Antistatica.

Die folgenden Beispiele illustrieren die Erfindung noch näher, ohne dass diese auf die Beispiele beschränkt werden soll. Die Temperaturen sind darin als °C angegeben.

**Beispiel 1**

**N(2,2,6,6-Tetramethylpiperidin-4-yl)-epsilon-Caprolactam**

a) Eine Lösung von 212 g (1,34 Mol) Triacetonamin (=2,2,6,6-Tetramethyl-4-piperidon) und 175 g (1,34 Mol) 6-Aminocapronsäure in 600 ml Wasser werden in einen 1-Liter-Autoklaven gefüllt, der mit Rühren, Wasserstoffeinleitung und Thermometer versehen ist. Nach Zusatz von 1,25 g eines Platinoxid-Katalysators wird die Lösung unter einem Wasserstoff-Druck von 3 Atmosphären (300 KPa) bei 25° hydriert bis die theoretische Menge Wasserstoff verbraucht ist.

Der Katalysator wird dann abfiltriert und das Filtrat eingedampft wobei man 285 g N-(2,2,6,6-Tetramethylpiperidin-4-yl)-6-amino-capronsäure als weisse Kristalle erhält, die bei 221 - 223° schmelzen.

b) In einem 500 ml-Kolben werden 100 g der gemäss a) hergestellten Säure unter einem Stickstoff-Strom 2 h auf 265° erhitzt, bis die Wasserabspaltung beendet ist. Das Reaktionsgefäss wird dann auf 110° gekühlt und 300 ml Heptan werden zugegeben. Die Mischung wird gerührt, bis sich der Grossteil des Rohproduktes gelöst hat. Vom Un gelösten wird heiss abfiltriert. Aus dem Filtrat scheiden sich beim Abkühlen weisse Kristalle von N-(2,2,6,6-Tetramethylpiperidin-4-yl)-epsilon-caprolactam ab, die bei 113 - 117° schmelzen.
Im Infrarot-Spektrum zeigt die Verbindung eine starke Carbamylabsorption bei 1626 $cm^{-1}$.

| | | | | |
|---|---|---|---|---|
| Analyse | ($C_{15}H_{28}N_2O$) | Ber. C 71,38 | H 11,18 | N 11,10 % |
| | | Gef. C 71,59 | H 11,28 | N 11,14 % |

**Beispiel 2**

**N-(2,2,6,6-Tetramethylpiperidin-4-yl)-zeta-önantholactam**

a) - Analog Beispiel 1a) wird unter Verwendung von 7-Aminoheptansäure die N-(2,2,6,6-Tetramethylpiperidin-4-yl)-7-aminoheptansäure hergestellt, die bis 162 - 165° schmilzt.

b) - Analog Beispiel 1 b) werden 50 g dieser Säure in Gegenwart von 0,1 g p-Toluolsulfansäure auf 300° erhitzt. Das Rohprodukt wird zuerst im Vakuum destilliert bei 155 - 160°/1 Pa und dann aus Heptan umkristallisiert. Das so erhaltene N-(2,2,6,6-Tetramethylpiperidin-4-yl)-zeta-önantholactam schmilzt bei 87 - 89°.

| | | | | |
|---|---|---|---|---|
| Analyse | ($C_{16}H_{30}N_2O$) | Ber. C 72,13 | H 11,35 | N 10,51 % |
| | | Gef. C 71,7 | H 11,1 | N 10,5 % |

**Beispiel 3**

**N-(1,2,2,6,6-Pentamethylpiperidin-4-yl)-epsilon-caprolactam**

Zu einer Lösung von 63 g (0,25 Mol) N-(2,2,6,6-Tetramethylpiperidin-4-yl)-epsilon-caprolactam, hergestellt gemäss Beispiel 1, in 150 ml Toluol wird eine Lösung von 28,5 ml (0,30 Mol) Dimethylsulfat in 100 ml Toluol unter Rühren zugegeben. Das Gemisch wird unter einem Rückflusskühler 1 h auf 80° erwärmt und dann 50 ml konzentrierte Ammoniaklösung zugegeben. Die Phasen werden getrennt, die Toluol-Phase wird über $K_2CO3$ getrocknet und im Vakuum eingedampft. Die im Titel genannte Verbindung hinterbleibt als weisses Pulver, das bei 76 - 79° schmilzt.

**Beispiel 4**

**N-(1-Allyl-2,2,6,6-tetramethylpiperidin-4-yl)-epsilon-Caprolactam**

In einer Lösung von 12,1 g (0,05 Mol) N-(2,2,6,6-Tetramethylpiperidin-4-yl)-epsilon-caprolactam in 75 ml Dimethylformamid werden 16,6 g (0,1 Mol) Natriumcarbonat suspendiert. Dazu gibt man 14,6 g (0,05 Mol) Allylbromid und erwärmt das Reaktionsgemisch unter Rühren 2 h auf 80°. Nach Filtration wird die Lösung im Vakuum eingedampft und der Rückstand im Vakuum destilliert bei 171 - 176° /100 Pa. Man erhält 7,6 g der Titelverbindung.

**Beispiel 5**

**N-(1-Propargyl-2,2,6,6-tetramethylpiperidin-4-yl)-epsilon-caprolactam**

Man arbeitet analog Beispiel 4 unter Verwendung der äquivalenten Menge an Propyrgylbromid anstelle von Allylbromid und erhält die Titelverbindung mit Sdp. 0,04 153 - 160° und Schmp. 105 - 109°.

| | | | | |
|---|---|---|---|---|
| Analyse | ($C_{18}H_{32}N_2O$) | Ber. C 74,4 | H 10,4 | N 9,6 % |
| | | Gef. C 74,3 | H 10,2 | N 9,7 % |

**Beispiel 6**

**N-(1-Benzyl-2,2,6,6-tetramethylpiperidin-4-yl)-epsilon-caprolactam**

Man arbeitet analog Beispiel 4 unter Verbindung der äquivalenten Menge Benzylchlorid anstelle von Allylbromid und erhält die Titelverbindung nach Umkristallisation aus Methanol/Wasser 4 : 1 mit einem Schmp. 126 - 127°.

| | | | | |
|---|---|---|---|---|
| Analyse | ($C_{22}H_{34}N_2O$) | Ber. C 77,2 | H 10,0 | N 8,2 % |
| | | Gef. C 77,4 | H 10,1 | H 8,0 % |

**Beispiel 7**

**N,N'-p-Xylylen-bis[4-(2-oxo-1-azepinyl)-2,2,6,6-tetramethylpiperidine]**

Man arbeitet wie in Beispiel 4 unter Verwendung der entsprechenden Menge p-Xylylendichlorid anstelle von Allylbromid. Das Rohprodukt schmilzt nach Umkristallisation aus Heptan/Methylenchlorid bei 280 - 283°.

| | | | | |
|---|---|---|---|---|
| Analyse | ($C_{38}H_{62}N_4O_2$) | Ber. C 75,2 | H 10,3 | N 9,2 % |
| | | Gef. C 75,0 | H 9,9 | N 9,1 % |

**Beispiel 8**

**N-(1-Cyano-2,2,6,6-tetramethylpiperidin-4-yl)-epsilon-caprolactam**

Man arbeitet wie in Beispiel 4 beschrieben unter Verwendung der äquivalenten Menge Chlorcyan anstelle von Allylbromid.

Die Titelverbindung schmilzt nach Umkristallisation aus Aceton/Wasser bei 168 - 169°.

| | | | | |
|---|---|---|---|---|
| Analyse | ($C_{16}H_{27}N_3O$) | Ber. C 69,3 | H 9,8 | N 15,1 % |
| | | Gef. C 69,1 | H 9,7 | N 15,0 % |

**Beispiel 9**

**N-(1-Acetyl-2,2,6,6-tetramethylpiperidin-4-yl)-epsilon-caprolactam**

Eine Lösung von 50,5 g (0,2 Mol) N-(2,2,6,6-Tetramethylpiperidin-4-yl)-apsilon-caprolactam (hergestellt gemäss Beispiel 1) in 102 g Essigsäureanhydrid wird mit 0,2 g $H_2SO_4$ versetzt und 4 h auf 120° erwärmt. Danach wird das überschüssige Essigsäureanhydrid in Vakuum abdestilliert. Der Rückstand wird in 200 ml Methylenchlorid gelöst, dreimal mit je 100 ml Wasser und einmal mit 10 %-iger $NaHCO_3$-Lösung gewaschen und über $Na_2SO_4$ getrocknet. Das nach Abdestillieren des Lösungsmittels hinterbleibende Produkt wird aus Toluol/Hexan 1 : 3 umkristallisiert. Man erhält 38,4 g der Titelverbindung, die bei 104 - 108° schmilzt.

**Beispiel 10**

**N-[1-(2-Hydroxyethyl)-2,2,6,6-tetramethylpiperidin-4-yl]-epsiloncaprolactam**

Ein Autoklav wird mit einer Lösung von 6,6 g (0,15 Mol) Ethylenoxid und 25,2 g (0,1 Mol) N-(2,2,6,6-Tetramethylpiperidin-4-yl)-epsilon-caprolactam (hergestellt gemäss Beispiel 1) in 100 ml Dimethylformamid beschickt. Nach Verschliessen wird der Autoklav 4 h auf 190° erhitzt, wobei der Druck von anfänglich 7 atm. (700 kPa) auf 4,9 atm. (490 kPa) abfällt. Das Reaktionsgemisch wird im Vakuum (50°/2mm) vom Lösungsmittel befreit. Der Rückstand wird aus 300 ml Heptan/Toluol 2 : 1 kristallisiert und man erhält so 13,7 g der Titelverbindung vom Schmp. 141 - 142°.

| | | | | |
|---|---|---|---|---|
| Analyse | $C_{17}H_{32}N_2O_2$) | Ber. C 68,9 | H 10,9 | N 9,4 % |
| | | Gef. C 68,8 | H 11,0 | N 9,2 % |

**Beispiel 11**

**Di-[2-[4-(2-oxo-1-azepinyl)-2,2,6,6-tetramethylpiperidin-4-yl]ethyl]-adipat**

Zu einer Lösung von 7 g (0,236 Mol) des Produktes von Beispiel 10 und 2 g (0,115 Mol) Domethyladipat in 50 ml Xylol

werden 50 mg $LiNH_2$ als Umesterungskatalysator zugegeben. Das Reaktionsgemisch wird 6 h auf 140° erwärmt unter ständigem Abdestillieren von Xylol. Der Rückstand wird aus Heptan/Toluol 2 : 1 zur Kristallisation gebracht. Man erhält so die Titelverbindung in einer Ausbeute von 4,9 g, die bei 159 - 160° schmilzt.

| | | | | |
|---|---|---|---|---|
| Analyse | ($C_{40}H_{70}N_4O_6$) | Ber. C 68,3 | H 10,0 | N 8,0 % |
| | | Gef. C 67,3 | H 10,2 | N 7,7 % |

**Beispiel 12**

**Di-[2-[4-(2-oxo-1-azepinyl)-2,2,6,6-tetramethylpiperidin-4-yl]ethyl]-sebacat**

Die Titelverbindung wird erhalten analog Beispiel 11 unter Verwendung von Dimethylsebacat anstelle von Dimethyladipat. Sie schmilzt nach Umkristallisation aus Heptan bei 104 - 111°.

| | | | | |
|---|---|---|---|---|
| Analyse | ($C_{44}N_{78}N_4O_6$) | Ber. C 69,6 | H 10,4 | N 7,4 % |
| | | Gef. C 69,7 | H 10,0 | N 7,2 % |

**Beispiel 13**

**2-[4-(2-Oxo-1-azepinyl)-2,2,6,6-tetramethylpiperidin-1-yl]ethyl-4-hydroxy-3,5-di-tert.-butylhydrocinnamat**

Die Titelverbindung wird analog Beispiel 11 unter Verwendung von Methyl-4-hydroxy-3,5-di-tert.-butylhydrocinnamat anstelle von Dimethyladipat. Nach Umkristallisation aus Xylol/Heptan 2 : 1 schmilzt das Produkt bei 159 - 161°.

| | | | | |
|---|---|---|---|---|
| Analyse | ($C_{34}H_{56}N_2O_4$) | Ber. C 73,3 | H 10,1 | N 5,0 % |
| | | Gef. C 73,1 | H 10,2 | N 5,1 % |

**Beispiel 14**

**N-(1-Oxyl-2,2,6,6-tetramethylpiperidin-4-yl)-epsilon-caprolactam**

Zu einer Lösung von 25,4 g (0,1 Mol) des Lactams aus Beispiel 1 in 200 ml Chloroform gibt man portionenweise im Laufe einer Stunde 38 g (0,22 Mol) m-Chlorperbenzoesäure. Das Reaktionsgemisch wird 18 h bei Raumtemperatur gerührt und dann von der gebildeten m-Chlorbenzoesäure abfiltriert. Das Filtrat wird nacheinander mit 1 n $H_2SO_4$, mit Wasser und mit 10 %-iger $NaHCO_3$-Lösung gewaschen und über $Na_2SO_4$ getrocknet. Die Lösung wird im Vakuum eingedampft und der Rückstand aus Toluol/Heptan umkristallisiert. Man erhält die Titelverbindung in Form von orangeroten Kristallen, die bei 158 - 164° schmecken. Das NMR-Spektrum bestätigt das Vorliegen einer paramagnetischen Verbindung (freies Radikal).

**Beispiel 15**

**N-(1-Hydroxy-2,2,6,6-tetramethylpiperidin-4-yl)-epsilon-caprolactam**

Eine Lösung von 26,7 g (0,1 Mol) des in Beispiel 16 erhaltenen Oxyls in 250 ml Dioxan wird zusammen mit 0,8 g Palladium auf Aktivkohle (5 % Pd) in einem Rührautoklaven bei Raumtemperatur unter 3 Atmosphären Wasserstoffdruck hydriert bis die theoretische Menge $H_2$ aufgenommen hat. Dann wird die Lösung auf 100° erwärmt und heiss vom Katalysator abfiltriert. Aus dem Filtrat kristallisiert das Produkt in der Kälte aus. Man erhält 18,7 g vom Schmp. 212 - 215°.

| | | | | |
|---|---|---|---|---|
| Analyse | ($C_{15}H_{28}N_2O_2$) | Ber. C 67,12 | H 10,52 | N 10,44 % |
| | | Gef. C 67,1 | H 10,0 | N 10,4 % |

**Beispiel 16**

**N-(1-Benzoyloxy-2,2,6,6-tetramethylpiperidin-4-yl)-epsilon-caprolactam**

Eine Lösung von 1,17 ml (0,01 Mol) Benzoylchlorid in 10 ml Tetrahydrofuran wird zu einer Lösung von 2,7 g (0,012 Mol) des Produktes aus Beispiel 15 und 1,4 ml Triethylamin in 75 ml Tetrahydrofuran unter Rühren langsam zugegeben. Das Reaktionsgemisch wird weitere 3 h bei 25° gerührt und dann 10 Minuten zum Rückfluss erhitzt. Das gebildete Triethylamin-hydrochlorid wird abfiltriert und die Lösung eingedampft. Der Rückstand wird aus Ethanol/Wasser umkristallisiert. Man erhält 2,4 g der Titelverbindung, die bei 154 - 156° schmilzt.
Das IR-Spektrum ($CH_2Cl_2$-Lösung zeigt zwei Carbonylbanden bei 1730 und 1621 $cm^{-1}$.

| | | | | |
|---|---|---|---|---|
| Analyse | ($C_{22}H_{32}N_2O_3$ | Ber. C 70,93 | H 8,66 | N 7,52 % |
| | | Gef. C 71,0 | H 8,7 | N 7,7 % |

**Beispiel 17**

**1,2,2,(6,6-Pentamethylpiperidin-4-yl-ester von 4-(2-Oxo-1-azepinyl)-2,2,6,6-tetramethylpiperidin-1-yl-essigsäure**

a) Eine Mischung von 50,5 g (0,2 Mol) des Lactams aus Beispiel 1, 49,0 g (0,4 Mol) Chloressigsäure-ethylester, 42,4 g (0,4 Mol) Natriumcarbonat und 100 ml Dimethylformamid (DMF) wird 7 h auf 130° erwärmt. Der überschüssige Chloressigsäureethylester wird zusammen mit 40 ml DMF im Vakuum abdestilliert. Das Reaktionsgemisch wird dann mit 500 ml Eiswasser verdünnt. Der gebildete Niederschlag wird abfiltriert, mit Wasser gewaschen und getrocknet. Umkristallisation aus Hexan ergibt 47,5 g 4-(2-Oxo-1-acepinyl)-2,2,6,6-tetramethylpiperidin-4-yl-essigsäureethylester, der bei 130 - 134° schmilzt. Das NMR-Spektrum dieser Verbindung stimmt mit der angenommenen Struktur überein.

b) Eine Lösung von 7,5 g (22 m Mol) des in a) erhaltenen Ethylesters und 4,2 g (24 m Mol) 1,2,2,6,6-Pentamethylpiperidin-4-o1 in 50 ml Xylol wird nach Zugabe von 0,1 g Lithiumamid eine Stunde auf 120° erwärmt. Das Reaktionsgefäss wird mit einem auf 150° gehaltenen Ölbad erwärmt, wobei ein Gemisch von Ethanol und Xylol langsam abdestilliert. Nach 5 h werden 4 Tropfen Essigsäure zugesetzt und der Rest des Xylols abdestilliert. Der Destillationsrückstand wird aus Heptan umkristallisiert und man erhält 6,1 g der Titelverbindung, die bei 166 - 168° schmilzt.
Die Struktur der Verbindung wird durch ihr NMR-Spektrum bestätigt.

| | | | | |
|---|---|---|---|---|
| Analyse | ($C_{27}H_{49}N_3O_3$) | Ber. C 69,9 | H 10,7 | N 9,1 % |
| | | Gef. C 69,9 | H 10,4 | N 9,0 % |

**Beispiel 18**

**1,6-Hexamethylenglykoldiester von 4-(2-Oxo-1-azepinyl)-2,2,6,6-tetramethylpiperidin-1-yl-essigsäure**

Nach der Verfahrensweise des Beispiels 17 b) erhält man aus dem Ethylester des Beispiels 17 a) und 1,6-Hexandiol von Molverhältnis 2 : 1 die Titelverbindung, die bei 147 - 149° schmilzt.

| | | | | |
|---|---|---|---|---|
| Analyse | ($C_{40}M_{70}N_4O_6$) | Ber. C 68,3 | H 10,0 | N 8,0 % |
| | | Gef. C 68,6 | H 9,8 | N 8,1 % |

**Beispiel 19**

**Bis-[4-(2-Oxo-1-azepinyl)-2,2,6,6-tetramethylpiperidin-1-ylacetat] des 1-(2-Hydroxyethyl)-2,2,6,6-tetramethylpiperidin-4-o1**

Diese Verbindung wird analog Beispiel 17 b hergestellt durch Reaktion von 1 Äquivalent 1-(2-Hydroxyethyl)-2,2,6,6-tetramethylpiperidin-4-o1 mit 2 Äquivalenten des Ethylesters aus Beispiel 17 a). Das Produkt schmilzt nach Umkristallisation aus Toluol bei 228 - 229°.

| | | | | |
|---|---|---|---|---|
| Analyse | ($C_{45}H_{79}N_5O_6$) | Ber. C 68,8 | H 10,1 | N 8,9 % |
| | | Gef. C 68,9 | H 9,9 | N 8,7 % |

**Beispiel 20**

**Licht-Stabilisierung von Polypropylen**

Polypropylen (Profax®, Hercules Corp.), das 0,1 % Calciumstearat aber kein Antioxydant enthält, wird mit jeweils 0,1 % der in der Tabelle 1 aufgeführten erfindungsgemässen Verbindungen vermischt. Die Mischung wird granuliert und bei einer Extrudertemperatur von 232° zu einem Band von 10,2 cm Breite und 0,127 mm Stärke extrudiert. Das Band wird in Längsrichtung zerschnitten in 6,4 mm breite Bändchen, welche über 107° heissen Godet-Walzen im Verhältnis 1 : 6 verstreckt werden. Man erhält verstreckte Farbbändchen von 0,05 mm Stärke.

Die Bändchen werden in einem Kohlebogenlampen-Bewitterungsgerät belichtet. Von Zeit zu Zeit wird die Reissfestigkeit der belichteten Faserbändchen gemessen. Die Belichtungszeit bis zu 50 % Abfall der Reissfestigkeit ist in Tabelle 1 aufgeführt.

**Tabelle 1**

| Zugesetzte Verbindung aus Beispiel Nr. (0,1 %) | Belichtungszeit (h) bis 50 % der ursprünglichen Reissfestigkeit |
|---|---|
| ohne | 230 |
| 1 | 2430 |
| 3 | 2445 |
| 6 | 1800 |
| 7 | 1810 |
| 12 | 1880 |
| 13 | 1490 |

**Beispiel 21**

**Stabilisierung eines Acrylharzlackes**

Zu einem Einbrennlack auf Basis eines hitzehärtbaren Acrylharzes, wie er für die Automobil-Lackierung üblich ist, werden die in Tabelle 2 angegebenen Stabilisatoren zugegeben. Der Lack wird auf Bleche aufgespritzt, die mit einem Primer grundiert sind. Die Proben werden 30 Minuten bei 120° gehärtet. Der Lackfilm hat eine Stärke von 0,038 mm.

Die Proben werden einem beschleunigten Bewitterungstest in einem QUV-Gerät unterworden, wobei die Proben abwechselnd 4 h bei 60° mit UV-Licht bestrahlt und 4 h bei 50° beregnet werden. Insgesamt werden die Proben 3540 h bewittert.

Vor und nach der Bewitterung wird der Oberflächenglanz bei einem Winkel von 20° nach ASTM D-523 in einem Reflexionsgoniometer gemessen, sowie die Abbildungsschärfe (Distructness of Image = D J) nach ASTM D-16 in einem Spektralphotometer gemessen. Der prozentuale Erhalt des Glanzes und der Bildschärfe wird in der Tabelle 2 aufgeführt.

**Tabelle 2**

| Stabilisation | Prozentuelle Erhaltung von 20°-Glanz | D J |
|---|---|---|
| keiner | 0[a)] | 0[a)] |
| 2 % Stabilisator A[b)] | 6 | 7 |
| 2 % Stabilisator A[b)] + 1 % Verbindung des Beispiels 1 | 28 | 33 |

a) Ohne Stabilisator war der Lack bereits nach 1100 h Bewitterung völlig zerstört
b) Stabilisator A: 2-[2-Hydroxy-3,5-di-(α,α-dimethylbenzyl)phenyl]-2H-benztriazol

**Patentansprüche**

1. Verbindung der Formel I oder II,

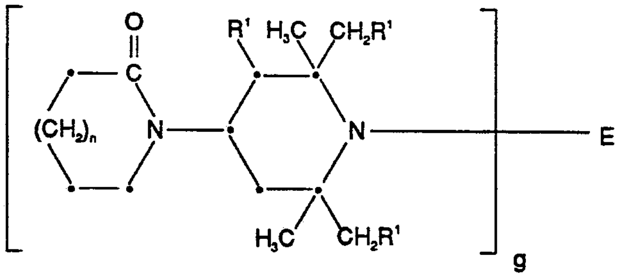

(I)

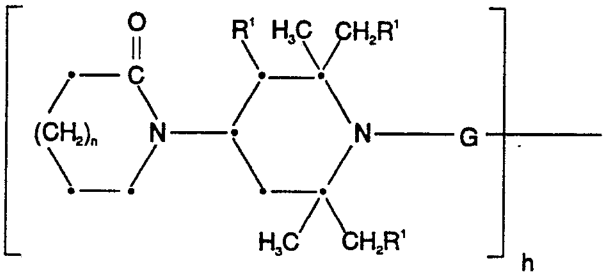

(II)

worin n 0, 1, 2 oder 3 ist, g 1 oder 2 ist, h eine Zahl von 1 bis 4 ist,

$R^1$ Wasserstoff oder $C_1$-$C_5$-Alkyl bedeutet,
E, wenn g 1 ist, H, O˙, OH, $C_1$-$C_{12}$-Alkyl, $C_3$-$C_8$-Alkenyl, Propargyl, Benzyl, CN, $C_2$-$C_4$-Hydroxyalkyl, $C_2$-$C_{10}$-Alkanoyl, $C_3$-$C_7$ Alkenoyl, Benzoyl, $C_2$-$C_8$-Alkoxy, $C_2$-$C_{10}$-Alkanoyloxy, $C_3$-$C_4$-Alkenoyloxy oder Benzoyloxy bedeutet, und wenn g 2 ist, unverzweigtes oder verzweigtes $C_2$-$C_6$-Alkylen, Phenylethylen(Styrolen), 2-Butenylen oder $C_8$-$C_{15}$ Phenylendialkylen bedeutet,

G ein Rest der Formel $-CH_2-CH(R^2)-O-$ oder $-CH_2-COO-$ ist, dessen Methylengruppe an den Piperidin-Stickstoff und dessen Sauerstoff an Q gebunden ist und worin $R^2$ Wasserstoff, Methyl, Ethyl oder Phenyl bedeutet, und
Q, wenn G $-CH_2-CH(R^3)-O-$ ist,

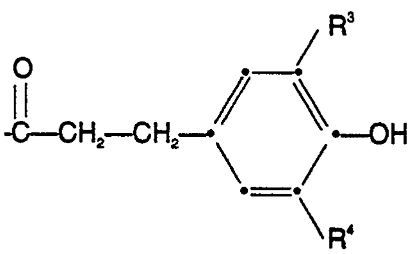

(III)

im Falle von h = 1 eine $C_2$-$C_{10}$-Alkanoylgruppe, Benzoylgruppe oder eine Gruppe der Formel III

bedeutet, worin $R^3$ $C_1$-$C_8$-Alkyl und $R^4$ verzweigtes $C_3$-$C_8$-Alkyl bedeuten,
im Falle von h = 2 -CO-, -COCO- oder $C_3$-$C_{12}$-Alkandicarbonyl bedeutet,
im Falle von h = 3 $C_6$-$C_{10}$-Alkantricarbonyl bedeutet und
im Falle von h = 4 $C_8$-$C_{12}$-Alkantetracarbonyl bedeutet und,
wenn G $-CH_2-COO-$ ist,

Q im Falle von h = 1 $C_1$-$C_{12}$-Alkyl oder eine Gruppe der Formel IV

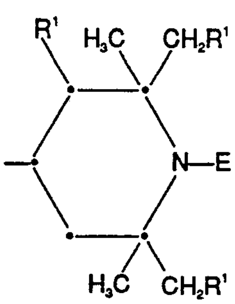

(IV)

bedeutet,

im Falle von h = 2 $C_2$-$C_{12}$-Alkylen, $C_6$-$C_8$-Cycloalkylen, 3-Oxapentamethylen, 1,4-Cyclohexylen-dimethylen oder eine Gruppe der Formel V

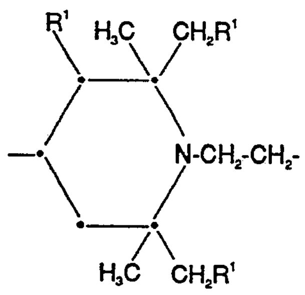

(V)

bedeutet,

im Falle von h = 3 $C_3$-$C_8$-Alkantriyl bedeutet und
im Falle von h = 4 $C_4$-$C_{10}$-Alkantetrayl bedeutet.

2. Verbindung gemäss Anspruch 1 der Formel I oder II, worin n 2 oder 3 ist.

3. Verbindung gemäss Anspruch 2, worin n 2 ist.

4. Verbindung gemäss Anspruch 1 der Formel I oder II, worin $R^1$ Wasserstoff ist.

5. Verbindung gemäss Anspruch 1 der Formel I, worin g = 1 ist und E Wasserstoff, O·, OH, $C_1$-$C_4$-Alkyl, Allyl, 2-Hydroxyethyl, Acetyl, Propargyl, Benzyl, Cyan, Benzoyl oder Benzoyloxy bedeutet oder g = 2 ist und E 1,2-Ethylen oder p-Xylylen bedeutet.

6. Verbindung gemäss Anspruch I der Formel II, worin G ein Rest der Formel $-CH_2CH_2-O-$ ist, h = 1 ist und Q Acetyl oder eine Gruppe der Formel III bedeutet, worin $R^3$ und $R^4$ tert.-Butyl sind, oder h = 2 ist und Q ein $C_6C_{10}$-Alkandicarbonylrest ist.

7. Verbindung gemäss Anspruch 1 der Formel II, worin G ein Rest der Formel $-CH_2-COO-$ ist, h = 1 ist und Q $C_1$-$C_4$-Alkyl oder eine Gruppe der Formel IV ist, oder h = 2 ist und Q $C_2$-$C_8$-Alkylen oder eine Gruppe der Formel V ist.

8. N-(2,2,6,6-Tetramethylpiperidin-4-yl)-epsilon-caprolactam, N-(1-Allyl-2,2,6,6-tetramethylpiperidin-4-yl)-epsilon-caprolactam Di-[2-[4-(2-oxo-1-azepinyl)-2,2,6,6-tetramethylpiperidin-1-yl]-ethyl]-sebacat, (1,2,2,6,6-Pentamethylpiperidin-4-yl)-4-(2-oxo-1-azepinyl)2,2,6,6-tetramethylpiperidin-1-ylacetat, N-(1-Acetyl-2,2,6,6-tetramethylpiperidin-4-yl)-epsilon-caprolactam oder N-(1-Hydroxy-2,2,6,6-tetramethylpiperidin-4-yl)epsiloncaprolactam als Verbindungen gemäss Anspruch 1.

9. Lichtempfindliches organisches Material stabilisiert durch Zusatz von 0,05 bis 5 Gew-% einer Verbindung der Formel I oder II des Anspruches 1.

10. Gemäss Anspruch 9 stabilisiertes organisches Polymer, insbesondere Polyolefin.

11. Verfahren zum Stabilisieren von lichtempfindlichen organischem Material durch Zusatz von 0,05 bis 5 Gew.-% einer Verbindung der Formel I oder II des Anspruches 1.

12. Verfahren zum Stabilisieren von Polyolefinen gemäss Anspruch 11.

13. Verwendung einer Verbindung der Formel I oder II des Anspruches 1 zum Stabilisieren von lichtempfindlichem Material.

**Claims**

1. A compound of the formula I or II,

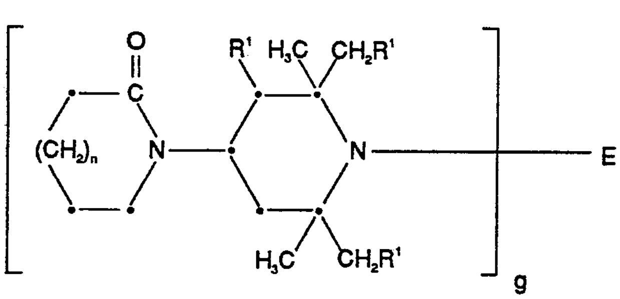

(I)

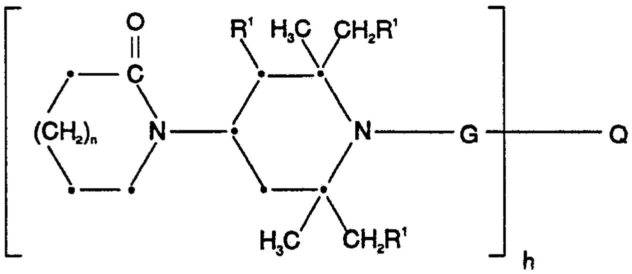

(II)

wherein n is 0, 1, 2 or 3, g is 1 or 2, h is a number from 1 to 4, $R^1$ is hydrogen or $C_1$-$C_5$alkyl, if g is 1, E is H, $O^{\cdot}$, OH, $C_1$-$C_{12}$alkyl, $C_3$-$C_8$alkenyl, propargyl, benzyl, CN, $C_2$-$C_4$hydroxyalkyl, $C_2$-$C_{10}$alkanoyl, $C_3$-$C_7$alkenoyl, benzoyl, $C_2$-$C_8$alkoxy, $C_2$-$C_{10}$alkanoyloxy, $C_3$-$C_4$alkenoyloxy or benzoyloxy, and if g is 2, E is unbranched or branched $C_2$-$C_6$alkylene, phenylethylene(styrolene), 2-butenylene or $C_8$-$C_{15}$phenylenedialkylene,

G is a radical of the formula $-CH_2-CH(R^2)-O-$ or $-CH_2-COO-$, the methylene group of which is bonded to the piperidine nitrogen and the oxygen of which is bonded to Q and wherein $R^2$ is hydrogen, methyl, ethyl or phenyl, and if G is $-CH_2-CH(R^3)-O-$, Q in the case of h = 1 is a $C_2$-$C_{10}$alkanoyl group, benzoyl group or a group of the formula III

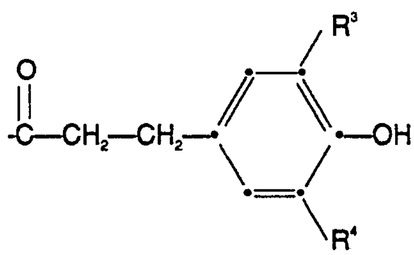

(III)

wherein $R^3$ is $C_1$-$C_8$alkyl and $R^4$ is branched $C_3$-$C_8$alkyl,
in the case of h = 2 Q is -CO-, -COCO- or $C_3$-$C_{12}$alkanedicarbonyl,
in the case of h = 3 Q is $C_6$-$C_{10}$alkanetricarbonyl and
in the case of h = 4 Q is $C_8$-$C_{12}$alkanetetracarbonyl and, when G is $-CH_2-COO-$,

Q in the case of h = 1 is $C_1$-$C_{12}$alkyl or a group of the formula IV

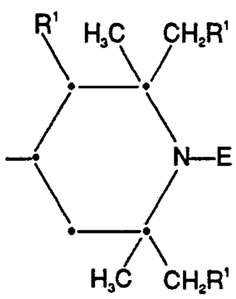

(IV)

in the case of h = 2 Q is $C_2$-$C_{12}$alkylene, $C_6$-$C_8$cycloalkylene, 3-oxapentamethylene, 1,4-cyclohexylenedimethylene or a group of the formula V

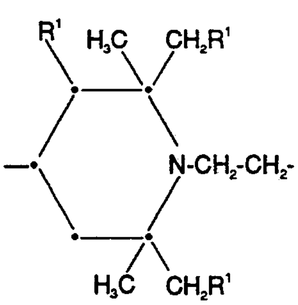

(V)

in the case of h = 3 Q is $C_3$-$C_8$alkanetriyl and
in the case of h = 4 Q is $C_4$-$C_{10}$alkanetetrayl.

2. A compound according to claim 1 of the formula I or II wherein n is 2 or 3.

3. A compound according to claim 2 wherein n is 2.

4. A compound according to claim 1 of the formula I or II wherein $R^1$ is hydrogen.

5. A compound according to claim 1 of the formula I wherein g = 1 and E is hydrogen, O·, OH, $C_1$-$C_4$alkyl, allyl, 2-hydroxyethyl, acetyl, propargyl, benzyl, cyano, benzoyl or benzoyloxy or g = 2 and E is 1,2-ethylene or p-xylylene.

6. A compound according to claim 1 of the formula II wherein G is a radical of the formula $-CH_2CH_2-O-$, h = 1 and Q is acetyl or a group of the formula III wherein $R^3$ and $R^4$ are tert.-butyl, or h = 2 and Q is a $C_6$-$C_{10}$alkanedicarbonyl radical.

7. A compound according to claim 1 of the formula II wherein G is a radical of the formula $-CH_2-COO-$, h = 1 and Q is $C_1$-$C_4$alkyl or a group of the formula IV, or h = 2 and Q is $C_2$-$C_8$alkylene or a group of the formula V.

8. N-(2,2,6,6-Tetramethylpiperidin-4-yl)-epsilon-caprolactam, N-(1-allyl-2,2,6,6-tetramethylpiperidin-4-yl)-epsilon-caprolactam di-[2-[4-(2-oxo-1-azepinyl)-2,2,6,6-tetramethylpiperidin-1-yl]-ethyl] sebacate, 1,2,2,6,6-pentamethylpiperidin-4-yl-4-(2-oxo-1-azepinyl)-2,2,6,6-tetramethylpiperidin-1-ylacetate, N-(1-acetyl-2,2,6,6-tetramethylpiperidin-4-yl)-epsilon-caprolactam or N-(1-hydroxy-2,2,6,6-tetramethylpiperidin-4-yl)-epsilon-caprolactam as compounds according to claim 1.

9. A light-sensitive organic material stabilized by the addition of from 0.05 to 5 % by weight of a compound of the formula I or II of claim 1.

10. An organic polymer, in particular a polyolefin, stabilized according to claim 9.

11. A method of stabilizing a light-sensitive organic material by the addition of from 0.05 to 5 % by weight of a compound of the formula I or II of claim 1.

12. A method of stabilizing a polyolefin according to claim 11.

13. Use of a compound of the formula I or II of claim 1 for stabilizing a light-sensitive material.

**Revendications**

1. Composé répondant à la formule I ou à la formule II:

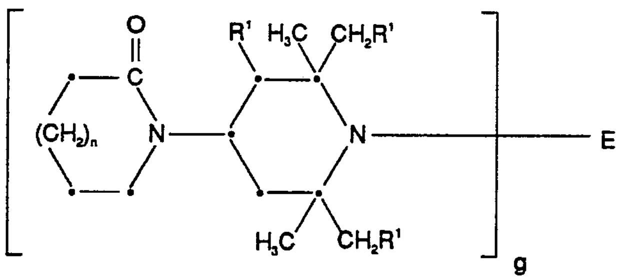

(I)

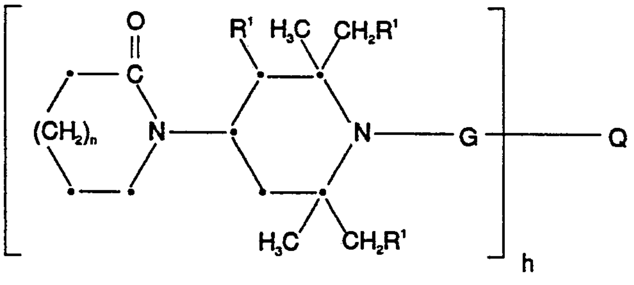

(II)

dans lesquelles:

n est égal à 0, à 1, à 2 ou à 3,
g est égal à 1 ou à 2,
h désigne un nombre de 1 à 4,
$R^1$ représente l'hydrogène ou un alkyle en $C_1$-$C_5$,
E représente:

- lorsque g est égal à 1 : H, O·, OH, un alkyle en $C_1$-$C_{12}$, un alcényle en $C_3$-$C_8$, un propargyle, un benzyle, CN, un hydroxyalkyle en $C_2$-$C_4$, un alcanoyle en $C_2$-$C_{10}$, un alcénoyle en $C_3$-$C_7$, un benzoyle, un alcoxy en $C_2$-$C_8$, un alcanoyloxy en $C_2$-$C_{10}$, un alcénoyloxy en $C_3$ ou $C_4$ ou un benzoyloxy, et
- lorsque g est égal à 2 : un alkylène en $C_2$-$C_6$ ramifié ou non, un phényléthylène (radical dérivant du styrène), un butène-2 ylène ou un phénylène-dialkylène en $C_8$-$C_{15}$,

G représente un radical $-CH_2-CH(R^2)-O-$ ou $-CH_2-COO-$ dont le radical méthylène est lié à l'azote pipéridinique et l'oxygène à Q et dont le symbole $R^2$ représente l'hydrogène ou un radical méthyle, éthyle ou phényle et
Q représente:

- lorsque G représente $-CH_2-CH(R^2)-O-$:
dans le cas où h est égal à 1, un radical alcanoyle en $C_2$-$C_{10}$, un radical benzoyle ou un radical répondant à la formule III:

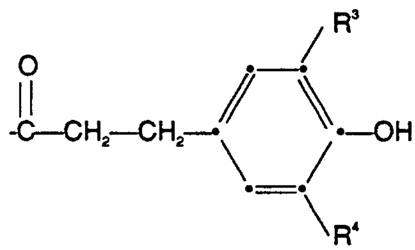

(III)

dans laquelle $R^3$ représente un alkyle en $C_1$-$C_8$ et $R^4$ un alkyle en $C_3$ - $C_8$ ramifié,
dans le cas où h est égal à 2, un radical -CO-, -COCO- ou alcane-dicarbonyle en $C_3$-$C_{12}$,
dans le cas où h est égal à 3, un radical alcane-tricarbonyle en $C_6$ - $C_{10}$ et
dans le cas où h est égal à 4, un radical alcane-tétracarbonyle en $C_8$-$C_{12}$, et

- lorsque G représente $-CH_2-COO-$: dans le cas où h est égal à 1, un alkyle en $C_1$-$C_{12}$ ou un radical de formule IV:

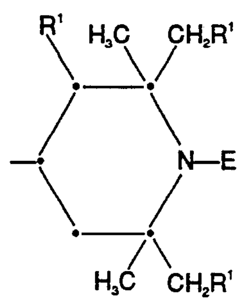

(IV)

dans le cas où h est égal à 2, un alkylène en $C_2$ - $C_{12}$, un cyclo-alkylène en $C_6$ - $C_8$, un oxa-3 pentaméthylène, un cyclohexylène-1,4 diméthylène ou un radical de formule V:

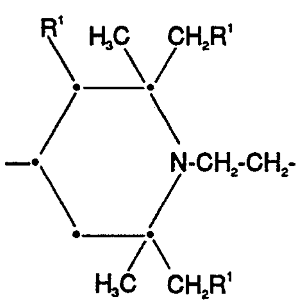

(V)

dans le cas où h est égal à 3, un radical alcane-triyle en $C_3$ - $C_8$ et
dans le cas où h est égal à 4, un radical alcane-tétrayle en $C_4$-$C_{10}$.

2. Composé selon la revendication 1 qui répond à la formule I ou II dans laquelle n est égal à 2 ou à 3

3. Composé selon la revendication 2 dans lequel n est égal à 2.

4. Composé selon la revendication 1 qui répond à la formule I ou II dans laquelle $R^1$ représente l'hydrogène.

5. Composé selon la revendication 1 qui répond à la formule I dans laquelle g est égal à 1 et E représente l'hydrogène, O·, OH, un alkyle en $C_1$-$C_4$, un allyle, un hydroxy-2 éthyle, un acétyle, un propargyle, un benzyle, un cyano, un benzoyle ou un benzoyloxy, ou g est égal à 2 et E représente un éthylène-1,2 ou un p-xylylène

6. Composé selon la revendication 1 qui répond à la formule II dans laquelle G représente un radical $-CH_2CH_2-O-$, h est égal à 1 et Q représente un radical acétyle ou un radical de formule III dans lequel $R^3$ et $R^4$ représentent chacun un radical tert-butyle, ou h est égal à 2 et Q représente un radical alcane-dicarbonyle en $C_6$ - $C_{10}$.

7. Composé selon la revendication 1 qui répond à la formule II dans laquelle G représente un radical $-CH_2-COO-$, h est égal à 1 et a représente un alkyle en $C_1$-$C_4$ ou un radical de formule IV, ou h est égal à 2 et Q représente un alkylène en $C_2$ - $C_8$ ou un radical de formule V.

8. Composé selon la revendication 1, en l'espèce:

le N-(tétraméthyl-2,2,6,6 pipéridyl-4)-ε-caprolactame,
le N-(allyl-1 tétraméthyl-2,2,6,6 pipéridyl)-4)-ε-caprolactame,
le sébaçate de bis-[((oxo-2 azépinyl-1)-4 tétraméthyl-2,2,6,6 pipéridyl-1)-2 éthyle],
l'[(oxo-2 azépinyl-1)-4 tétraméthyl-2,2,6,6 pipéridyl-1]-acétate de pentaméthyl -1,2,2,6,6 pipéridyle-4,
le N-(acétyl-1 tétraméthyl-2,2,6,6 pipéridyl-4)-ε-caprolactame ou
le N-(hydroxy-1 tétraméthyl-2,2,6,6 pipéridyl-4)-ε-caprolactame.

9. Matière organique sensible à la lumière qui a été stabilisée par addition de 0,05 à 5 % en poids d'un composé de formule I ou II selon la revendication 1.

10. Polymère organique, en particulier polyoléfine, qui a été stabilisé selon la revendication 9.

11. Procédé pour stabiliser une matière organique sensible à la lumière, procédé selon lequel on ajoute de 0,05 à 5 % en poids d'un composé de formule I ou II selon la revendication 1.

12. Procédé pour stabiliser des polyoléfines selon la revendication 11.

13. Application d'un composé de formule I ou II selon la revendication 1 à la stabilisation d'une matière sensible à la lumière.